# FASCICULE DE BREVET EUROPEEN

(11) **EP 1 433 998 B1**
(45) Date de publication et mention de la délivrance du brevet: **11.05.2005**
(21) Numéro de dépôt: 03293099.2
(22) Date de dépôt: 10.12.2003
(51) Int. Cl.: F21S 8/00, G01J 1/32

(54) **Dispositif d'éclairage et son utilisation**
Beleuchtungsvorrichtung und dessen Verwendung
Lighting device and use thereof

(30) Priorité: 24.12.2002 FR 0216676
(43) Date de publication de la demande: 30.06.2004
(73) Titulaire: ALM, 45160 Ardon (FR)
(72) Inventeur: Thibaud, Philippe, 45100 Orleans (FR); Monnot, Jérôme, 45240 Menestreau en Villette (FR); Bouillhol, Sébastien, 91160 Saulx les Chartreux (FR); Fichot, Yves, 45000 Orleans (FR)
(74) Mandataire: Domenego, Bertrand

(56) Documents cités:
- WO-A-99/22224
- DE-A- 10 034 594
- US-A- 4 288 844

## Description

La présente invention concerne un dispositif d'éclairage d'un champ d'éclairage, notamment d'un champ d'intervention d'un médecin, du type comportant une source de lumière comprenant un premier et un second modules d'éclairage, ces modules d'éclairage tant adaptés pour éclairer respectivement un premier et un second domaines d'éclairage du champ d'éclairage, lesdits domaines d'éclairage se chevauchant au moins partiellement en formant une zone de chevauchement, et chaque module d'éclairage définissant un volume de chevauchement s'étendant entre la surface du module d'éclairage émettant de la lumière vers la zone de chevauchement et cette zone de chevauchement.

Elle s'applique notamment aux dispositifs d'éclairage de champs d'opération dans des salles d'opération de chirurgie.

On connaît des dispositifs d'éclairage dans des salles d'opération. De tels dispositifs comprennent une source de lumière dont le faisceau lumineux est dirigé vers le champ d'opération.

Lorsqu'un obstacle, tel que la tête d'un médecin, entre dans la marche des rayons de la source de lumière vers le champ d'éclairage, une tâche d'ombre est formée, ce qui diminue la qualité d'éclairage et est peu ergonomique.

La présente invention a pour but de pallier cet inconvénient, et de proposer un dispositif d'éclairage qui ait une ergonomie améliorée.

A cet effet l'invention a pour objet un dispositif du type précité, caractérisé en ce qu'il comprend des moyens de détection adaptés pour détecter une zone de moindre éclairement dans la zone de chevauchement, et des moyens de commande adaptés pour augmenter le flux lumineux dudit second module d'éclairage lorsque les moyens de détection détectent une zone de moindre éclairement.

Selon d'autres modes de réalisation, l'invention comporte l'une ou plusieurs des caractéristiques suivantes :
- la source de lumière est adaptée pour éclairer la zone de chevauchement avec un éclairement déterminé et les moyens de commande sont adaptés pour augmenter le flux lumineux dudit second module d'éclairage de telle sorte que l'éclairement de la zone de chevauchement est égal à l'éclairement déterminé ;
- les moyens de détection sont adaptés pour détecter une zone d'ombre qui est créée par un obstacle se trouvant dans le volume de chevauchement dudit premier module d'éclairage et qui est située au moins partiellement à l'intérieur de la zone de chevauchement, en les moyens de commande sont adaptés pour augmenter le flux lumineux dudit second module d'éclairage d'un taux déterminé, et le taux déterminé est égal au rapport de la surface de la zone d'ombre à la surface de la zone de chevauchement ;
- les moyens de commande comprennent des moyens de diminution du flux lumineux, adaptés pour diminuer le flux lumineux dudit premier module d'éclairage lors de la constatation d'une zone de moindre éclairement par les moyens de détection ;
- lesdits premier et second modules d'éclairage sont chacun adaptés pour émettre de la lumière d'une composition spectrale identique ;
- au moins l'un des modules d'éclairage comprend une diode électroluminescente ;
- au moins l'un des modules d'éclairage comporte plusieurs diodes électro-luminescentes dont au moins deux diodes électro-luminescentes sont adaptées pour émettre de la lumière de couleur différente, et les moyens de commande comprennent une unité de commande associée à chaque module d'éclairage, cette unité de commande étant adaptée pour régler le flux lumineux individuel de la lumière émise par chacune des diodes électroluminescentes du module d'éclairage en fonction d'une valeur de consigne donnée représentant une couleur et une intensité ;
- la source de lumière comprend une pluralité de modules d'éclairage comprenant chacune plusieurs diodes électroluminescentes, et les moyens de commande comprennent une unité centrale adaptée pour générer des valeurs individuelles de consigne représentant une couleur et une intensité de lumière pour chacun des modules d'éclairage, et chaque unité de commande est reliée à l'unité centrale de façon à pouvoir recevoir les valeurs de consigne de couleur et d'intensité ;
- au moins un module d'éclairage comprend des moyens de détection d'une diode électroluminescente défectueuse, et il comprend en outre des moyens de compensation d'un défaut d'une diode électroluminescente défectueuse ; et
- les moyens de compensation comprennent des moyens adaptés pour diminuer le flux lumineux des diodes électroluminescentes émettant de la lumière d'une couleur autre que celle de la diode électroluminescente défectueuse ;
- le module d'éclairage comprend au moins une autre diode électroluminescente, d'une couleur identique à celle de la diode électroluminescente défectueuse, et les moyens de compensation sont adaptés pour, augmenter le flux lumineux de la diode électroluminescente ayant la même couleur que la diode électroluminescente défectueuse ;
- l'indice de rendu couleur de la source de lumière est d'au moins 90.

L'invention a en outre pour objet une utilisation d'un dispositif tel que précité pour éclairer un champ d'intervention d'un médecin.

L'invention sera mieux comprise à la lecture de la description qui va suivre, donnée uniquement à titre d'exemple et faite en se référant aux dessins annexés, sur lesquels :
- la Figure 1 est une vue schématique de côté d'une installation d'éclairage selon l'invention;
- la Figure 2 est une vue d'un ensemble d'éléments d'éclairage suivant la ligne II-II de la Figure 1;
- la Figure 3 est une vue d'un élément d'éclairage selon l'invention ; et
- la Figure 4 est une vue d'une partie du champ d'éclairage suivant la ligne IV-IV de la Figure 1.

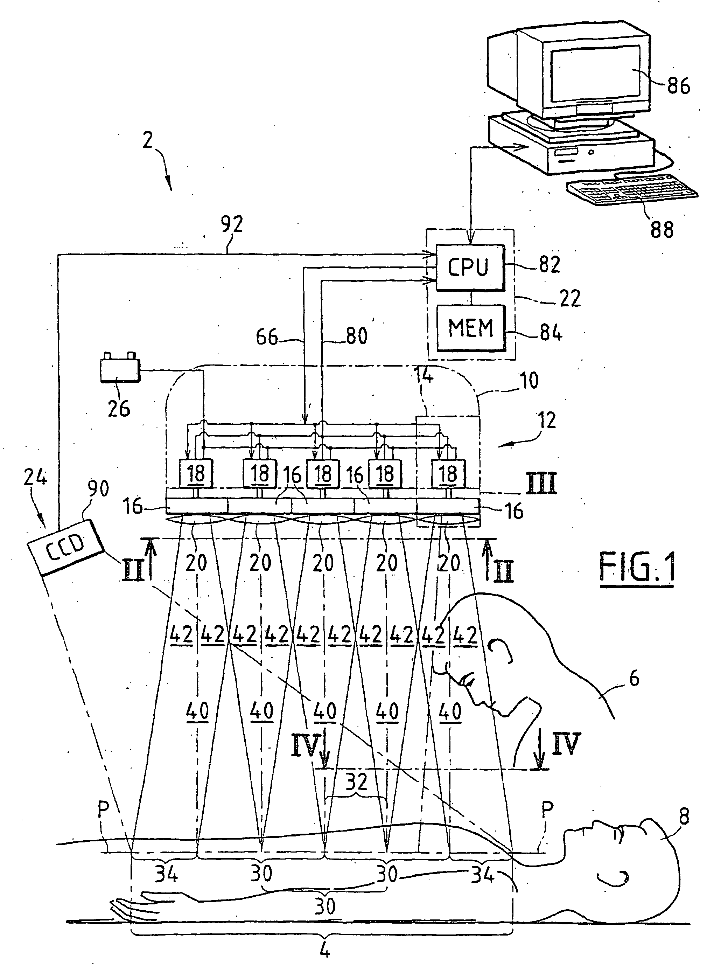

Sur la Figure 1 est représentée une installation d'éclairage selon l'invention, désignée par la référence générale 2.

L'installation est destinée à éclairer un champ d'éclairage 4 s'étendant suivant un plan d'éclairage horizontal P-P. En l'occurrence le champ d'éclairage 4 est le champ d'intervention d'un médecin 6 sur un patient 8 dans une salle d'opération médicale.

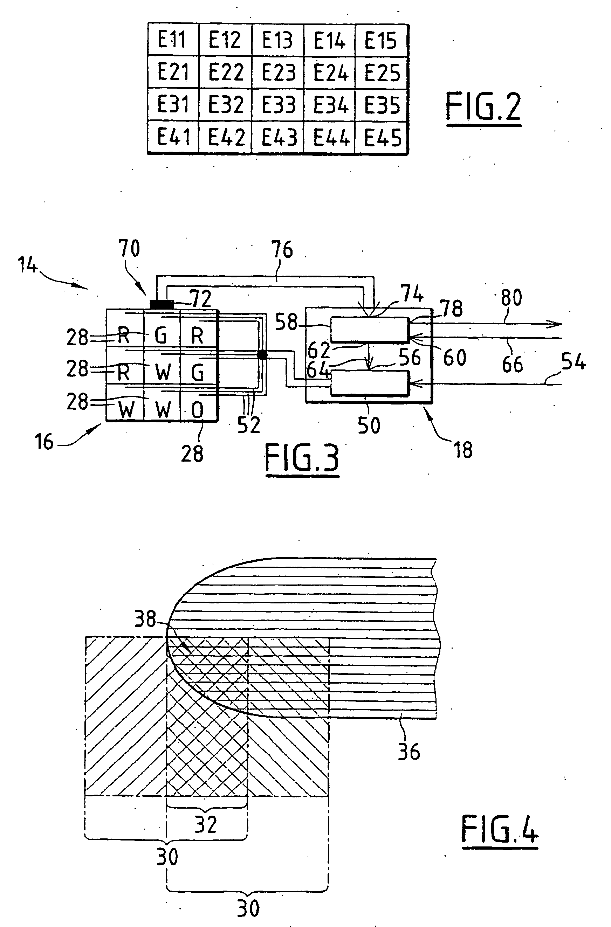

L'installation 2 comprend un boîtier 10 dans lequel est disposée une source de lumière 12 formée d'une pluralité d'éléments d'éclairage 14. Dans le cas présent, l'installation 2 comprend une matrice de vingt éléments d'éclairage 14, agencés en cinq colonnes à quatre lignes, comme représenté sur la Figure 2. Les cinq éléments d'éclairage 14 de la première ligne sont visibles sur la Figure 1.

Chaque élément d'éclairage 14 comprend un module d'éclairage 16, une unité de commande 18 associée au module d'éclairage 16 et des éléments optiques 20.

L'installation 2 comprend en outre une unité centrale 22, des moyens de détection 24 d'un obstacle et une source d'électricité 26.

Comme illustré sur la Figure 3, chaque module d'éclairage 16 comprend neuf diodes électroluminescentes 28 (LED) qui sont disposés suivant une matrice 3x3. Les modules d'éclairage 16 sont disposés dans un plan E-E parallèle au plan P-P. La direction d'émission de lumière de chaque LED 28 est perpendiculaire au plan E-E. Chaque module d'éclairage 16 comprend au moins une LED rouge (R), verte (G) et orange (O), ainsi qu'une LED blanche (W). Dans l'exemple représenté, chaque module d'éclairage 16 comprend trois LEDs 28 rouges, deux LEDs 28 vertes et une LED 28 orange, ainsi que trois LEDs 28 blanches. Le rapport LED rouge:LED vert:LED orange:LED blanche est donc 3:2:1:3.

Le maximum du spectre d'émission de chacune des LEDs 28 est de préférence situé dans les plages suivantes :
LED rouge : 645 nm à 660 nm
LED verte : 513 nm à 528 nm
LED orange: 592 nm à 597 nm
LED blanche : 460 nm à 470 nm.

De préférence, les modules d'éclairage 16 sont tous identiques. Les LEDs 28 du module d'éclairage 16 sont choisies de telle sorte que l'indice de rendu couleur (CRI) d'un module d'éclairage 16 est supérieur à 90, afin d'assurer que les couleurs de tissu du patient 8 soient représentées d'une manière proche de la réalité.

Chaque module d'éclairage 16 est adapté pour éclairer un domaine d'éclairage 30 déterminé, qui est situé à l'intérieur du champ d'éclairage 4 et qui est constitué d'une partie de la surface du champ d'éclairage 4.

Les modules d'éclairage 16 sont agencés de telle sorte que les domaines d'éclairage 30 de deux modules d'éclairage 16 adjacents se chevauchent partiellement en formant une zone de chevauchement 32 (voir Figure 4). Deux domaines d'éclairage 30 adjacents se chevauchent de la moitié de leurs surfaces, de telle sorte que chaque emplacement du champ d'éclairage 4 reçoit de la lumière de deux modules d'éclairage 16, à l'exception d'une zone marginale 34 du champ d'éclairage de largeur d'une moitié des dimensions d'un domaine d'éclairage 30 (voir Figure 1).

Sur la Figure 4 sont illustrés à titre d'exemple les domaines d'éclairage 30 de deux modules d'éclairage 16, formant une zone de chevauchement 32.

Sur la Figure 4 est également représenté un domaine d'ombre 36 créé par la tête du médecin 6. Le domaine d'ombre 36 s'étend dans la zone de chevauchement 32 en formant une zone d'ombre 38.

Chaque module d'éclairage 16 est adapté pour émettre un volume de lumière 40 (voir Figure 1), en l'occurrence un tronc de pyramide, qui s'étend entre la surface émettrice du module d'éclairage 16 qui émet de la lumière vers le domaine d'éclairage et le domaine d'éclairage 30 correspondant. Chaque volume de lumière 40 définit également au moins un volume de chevauchement 42 s'étendant entre la surface émettrice du module d'éclairage 16 qui émet de la lumière vers la zone de chevauchement et la zone de chevauchement 32 correspondante.

Les éléments optiques 20 sont disposés dans la marche de rayons de chaque module d'éclairage 16. Ces éléments optiques 20 sont par exemple des lentilles et sont adaptés pour focaliser la lumière émise vers le plan d'éclairage P-P, de telle sorte que la lumière émise par chaque module d'éclairage 16 ait une composition spectrale uniforme, dans le plan P-P.

Comme représenté sur la Figure 3, chacune des unités de commande 18 comprend un régulateur de courant 50 qui est relié individuellement à chacune des LEDs 28 du module d'éclairage 16 associé au moyen de neuf lignes d'alimentation 52. Le régulateur de courant 50 est connecté à la source d'électricité 26 par une ligne d'alimentation 54. Une entrée 56 portée par le régulateur de courant 50 permet de régler individuellement le courant passant par chacune des LEDs 28 et de déterminer ainsi le flux lumineux émis par chacune de ces LEDs 28.

L'unité de commande 18 comporte par ailleurs un bloc de calcul 58, qui peut être par exemple un microcontrôleur, ayant une entrée de couleur et d'intensité 60 pour des valeurs de consigne de couleur et d'intensité de la lumière à émettre par le module d'éclairage 16. Une sortie 62 de ce bloc de calcul 58 est reliée à l'entrée 56 du régulateur de courant 50 par une ligne de connexion. Le bloc de calcul 58 est adapté pour calculer à partir des valeurs de consigne une intensité de courant de consigne pour chacune des LEDs 28 de ce module d'éclairage 16 et de transmettre ces intensités individuellement au régulateur de courant 50.

L'entrée 60 est reliée par une ligne de connexion 66 à l'unité centrale 22. Chacune des unités de commande 18 est ainsi adaptée pour régler le flux lumineux de la lumière émise par les neuf LEDs 28 du module d'éclairage associé en fonction de données émises par l'unité centrale 22.

L'élément d'éclairage 14 comprend par ailleurs des moyens 70 de détection d'un défaut, adaptés pour détecter une LED 28 défectueuse du module d'éclairage 16 associé. Ces moyens 70 sont par exemple un bloc 72 de capteurs de courant qui sont adaptés pour détecter le courant passant effectivement à travers chacune des LEDs 28 et reliés à une entrée 74 du bloc de calcul 58 par une ligne de connexion 76.

Le bloc de calcul 58 comprend en outre une sortie 78 reliée à l'unité centrale par une ligne de connexion 80.

L'unité centrale 22 est constituée par un ordinateur comprenant un processeur 82 et une mémoire 84 qui stocke un logiciel d'exploitation de l'installation d'éclairage 2, ainsi que des paramètres d'exploitation. L'ordinateur est relié à des moyens d'affichage 86, sous forme d'un écran, et des moyens d'entrée de données 88, par exemple sous forme d'un clavier.

Les moyens d'entrée 88 permettent de saisir par un utilisateur des valeurs de consigne représentant la couleur globale de la lumière d'éclairage et l'intensité globale de la lumière ou représentant la température de couleur.

Les moyens 24 de détection d'un obstacle comprennent une camera CCD 90, qui est reliée à l'ordinateur par une ligne de connexion 92. En variante, tout autre moyen de détection de présence d'obstacle peut être utilisé, par exemple un détecteur de proximité. Le champ de vision de cette camera CCD 90 est dirigé vers le champ d'éclairage 4, et permet de capter une image de celui-ci. La ligne de connexion 92 est adaptée pour transmettre l'image captée à l'ordinateur.

L'installation selon l'invention fonctionne de la façon suivante.

Initialement, l'utilisateur saisit des valeurs qui représentent la couleur et l'intensité souhaitées de la lumière par le clavier. Ces valeurs sont stockées dans la mémoire 84 par le processeur 82 en tant que valeur de consigne globale d'intensité et valeur de consigne globale de couleur.

Lorsqu'aucun obstacle se trouve dans le volume d'éclairage 40, des valeurs de consigne individuelles sont transmises à chacune des unités de commande 18 sans modification. En d'autres termes, chacun des éléments d'éclairage 14 reçoit les mêmes valeurs de consigne de température de couleur, d'intensité et de répartition des intensités relatives de chacun des LED's 28.

Les valeurs de consigne de couleur et d'intensité sont alors converties par le bloc de calcul 58 en des valeurs d'intensité de courant pour chacune des LEDs 28.

Etant donné que les valeurs de consigne sont identiques pour chacun des éléments d'éclairage 14, chacun de ces éléments 14 émet un flux lumineux identique. Le champ d'éclairage 4 est donc éclairé uniformément.

L'image captée par la caméra CCD 90 présente alors une intensité sensiblement uniforme.

Dans le cas où un détecteur de proximité est utilisé au lieu de la caméra CCD, ce détecteur évalue la surface de l'obstacle.

Lorsqu'un obstacle, tel que la tête du médecin 6, entre dans le volume de chevauchement 42 de l'un des modules d'éclairage 16, ceci crée une zone d'ombre 38 dans le champ d'éclairage 4, ce qui nuit à la qualité d'éclairage.

L'image captée par la camera CCD 90 diminue donc d'intensité dans une zone correspondant à la zone d'ombre 38. L'unité centrale 22 évalue l'image captée par la caméra CCD 90 et constate cette diminution d'intensité. Ensuite, l'unité centrale détermine les modules d'éclairage 16 qui contribuent à l'éclairage de la zone de chevauchement 32 dans laquelle est située la zone d'ombre 38, ainsi que le module d'éclairage 16 dans le volume- d'éclairage 40 duquel est situé l'obstacle.

A cet effet, l'image captée par la caméra CCD 90 est divisée en une multitude d'éléments d'image, dont chacun correspond à un module d'éclairage 16.

Suite à cette constatation d'obstacle, l'unité centrale 22 augmente progressivement la valeur de consigne d'intensité des modules d'éclairage 16 qui contribuent à l'éclairage de la zone de chevauchement 32 rendue plus foncée, à l'exception des modules d'éclairage 16 dans le volume d'éclairage 40 desquels est situé l'obstacle. Cette augmentation est poursuivie jusqu'à ce que le flux lumineux soit suffisamment important pour que l'intensité de la zone d'ombre 32 ait atteint l'intensité correspondant à l'intensité qui correspond à la valeur de consigne d'intensité.

En variante, la valeur de consigne d'intensité est augmentée d'un taux déterminé, ce taux déterminé étant égal au rapport de la surface de la zone d'ombre 38 à la surface de la zone de chevauchement 32.

Ainsi l'intensité de lumière dans la zone d'ombre 38 est augmentée et la visibilité améliorée.

Lorsqu'une des LEDs 28 d'un module d'éclairage 16 est défectueuse, l'unité de commande 18 constate ce défaut par l'intermédiaire du capteur 72 et augmente le courant passant par les autres LEDs 28 de même couleur de ce module, afin de compenser la diminution du flux de lumière de cette couleur émise par de module d'éclairage 16.

En variante, l'unité de commande 18 diminue l'intensité de la lumière émise par des LEDs 28 ayant une couleur autre que celle de la LED 28 défectueuse, jusqu'à ce que la couleur de la lumière émise par ce module correspond à la couleur de consigne. Ainsi des perturbations de l'éclairage dues à une modification locale de la couleur d'éclairage sont évitées.

En variante, l'unité centrale 22 diminue également la valeur de consigne d'intensité de l'élément d'éclairage 14 dans le volume de chevauchement 42 duquel est situé l'obstacle. Ainsi, la consommation d'énergie de l'installation est diminuée.

Selon une variante non représentée, deux modules d'éclairage 16 qui sont adaptés pour éclairer une zone de chevauchement 32 commune sont séparés par au moins un module d'éclairage 16 adapté pour éclairer une autre zone de chevauchement 32. Ainsi, les deux modules 16 sont espacés de telle sorte que les volumes d'éclairage 40 ne se chevauchent que peu. Un obstacle qui entre dans le volume de chevauchement 42 d'un des modules d'éclairage 16 n'entre donc que peu dans le volume d'éclairage 40 de l'autre des modules d'éclairage 16. Ce dernier module 16 peut donc mieux assurer l'éclairage de la zone de chevauchement 32.

## Revendications

1. Dispositif d'éclairage d'un champ d'éclairage (4), notamment d'un champ d'intervention d'un médecin, du type comportant une source de lumière (12) comprenant un premier et un second modules d'éclairage (16), ces modules d'éclairage (16) étant adaptés pour éclairer respectivement un premier et un second domaines d'éclairage (30) du champ d'éclairage (4), lesdits domaines d'éclairage (30) se chevauchant au moins partiellement en formant une zone de chevauchement (32), et chaque module d'éclairage (16) définissant un volume de chevauchement (42) s'étendant entre la surface du module d'éclairage (16) émettant de la lumière vers la zone de chevauchement (32) et cette zone de chevauchement (32),
**caractérisé en ce qu'**il comprend :
- des moyens de détection (24) adaptés pour détecter une zone de moindre éclairement dans la zone de chevauchement (32), et
- des moyens de commande (18, 22) adaptés pour augmenter le flux lumineux dudit second module d'éclairage (16) lorsque les moyens de détection (24) détectent une zone de moindre éclairement.

2. Dispositif d'éclairage suivant la revendication 1, **caractérisé en ce que** la source de lumière (12) est adaptée pour éclairer la zone de chevauchement (32) avec un éclairement déterminé, et **en ce que** les moyens de commande (18, 22) sont adaptés pour augmenter le flux lumineux dudit second module d'éclairage (16) de telle sorte que l'éclairement de la zone de chevauchement (32) est égal à l'éclairement déterminé.

3. Dispositif d'éclairage suivant la revendication 1 ou 2, **caractérisé en ce que** les moyens de détection (24) sont adaptés pour détecter une zone d'ombre (38) qui est créée par un obstacle se trouvant dans le volume de chevauchement (42) dudit premier module d'éclairage (16) et qui est située au moins partiellement à l'intérieur de la zone de chevauchement (32), **en ce que** les moyens de commande (18, 22) sont adaptés pour augmenter le flux lumineux dudit second module d'éclairage (16) d'un taux déterminé, et **en ce que** le taux déterminé est égal au rapport de la surface de la zone d'ombre (38) à la surface de la zone de chevauchement (32).

4. Dispositif d'éclairage suivant l'une quelconque des revendications 1 à 3, **caractérisé en ce que** les moyens de commande (22) comprennent des moyens de diminution du flux lumineux, adaptés pour diminuer le flux lumineux dudit premier module d'éclairage (16) lors de la constatation d'une zone de moindre éclairement par les moyens de détection (24).

5. Dispositif d'éclairage suivant l'une quelconque des revendications précédentes, **caractérisé en ce que** lesdits premier et second modules d'éclairage (16) sont chacun adaptés pour émettre de la lumière d'une composition spectrale identique.

6. Dispositif d'éclairage suivant l'une quelconque des revendications 1 à 5, **caractérisé en ce qu'**au moins l'un des modules d'éclairage (16) comprend une diode électroluminescente (28).

7. Dispositif d'éclairage suivant la revendication 6, **caractérisé en ce qu'**au moins l'un des modules d'éclairage (16) comporte plusieurs diodes électroluminescentes (28) dont au moins deux diodes électroluminescentes (28) sont adaptées pour émettre de la lumière de couleur différente, et **en ce que** les moyens de commande comprennent une unité de commande (18) associée à chaque module d'éclairage (16), cette unité de commande (18) étant adaptée pour régler le flux lumineux individuel de la lumière émise par chacune des diodes électroluminescentes (28) du module d'éclairage (16) en fonction d'une valeur de consigne donnée représentant une couleur et une intensité.

8. Dispositif d'éclairage suivant la revendication 7, **caractérisé en ce que** la source de lumière (12) comprend une pluralité de modules d'éclairage (16) comprenant chacune plusieurs diodes électroluminescentes (28), **en ce que** les moyens de commande comprennent une unité centrale (22) adaptée pour générer des valeurs individuelles de consigne représentant une couleur et une intensité de lumière pour chacun des modules d'éclairage (16), et **en ce que** chaque unité de commande (18) est reliée à l'unité centrale (22) de façon à pouvoir recevoir les valeurs de consigne de couleur et d'intensité.

9. Dispositif d'éclairage suivant la revendication 8, **caractérisé en ce qu'**au moins un module d'éclairage (16) comprend des moyens de détection (70) d'une diode électroluminescente défectueuse (28), et **en ce qu'**il comprend en outre des moyens de compensation d'un défaut d'une diode électroluminescente défectueuse.

10. Dispositif d'éclairage suivant la revendication 9, **caractérisé en ce que** les moyens de compensation comprennent des moyens adaptés pour diminuer le flux lumineux des diodes électroluminescentes (28) émettant de la lumière d'une couleur autre que celle de la diode électroluminescente défectueuse (28).

11. Dispositif d'éclairage suivant la revendication 9, caractérisé en ce le module d'éclairage comprend au moins une autre diode électroluminescente (28), d'une couleur identique à celle de la diode électroluminescente défectueuse, et en ce que les moyens de compensation sont adaptés pour augmenter le flux lumineux de la diode électroluminescente ayant la même couleur que la diode électroluminescente défectueuse (28).

12. Dispositif d'éclairage suivant l'une quelconque des revendications 1 à 11, **caractérisé en ce que** l'indice de rendu couleur (CRI) de la source de lumière (12) est d'au moins 90.

13. Utilisation d'un dispositif d'éclairage suivant l'une quelconque des revendications précédentes pour éclairer un champ d'intervention d'un médecin.

## Patentansprüche

1. Beleuchtungsvorrichtung für ein Beleuchtungsfeld (4), insbesondere ein Operationsfeld eines Arztes, mit einer Lichtquelle (12), die einen ersten und einen zweiten Beleuchtungsmodul (16) umfasst, welche Beleuchtungsmodule (16) jeweils einen ersten und einen zweiten Beleuchtungsbereich (30) des Beleuchtungsfeldes (4) beleuchten können, welche Beleuchtungsbereiche (30) sich wenigstens teilweise so überlappen, dass eine Überlappungszone (32) gebildet ist, wobei jeder Beleuchtungsmodul (16) ein Überlappungsvolumen (42) festlegt, das sich zwischen der Oberfläche des Beleuchtungsmoduls (16), die das Licht zur Überlappungszone (32) ausgibt, und dieser Überlappungszone (32) erstreckt,
**dadurch gekennzeichnet, dass** sie
- Erfassungseinrichtungen (24), die eine Zone geringer Beleuchtung in der Überlappungszone (32) erfassen können, und
- Steuereinrichtungen (18, 22) umfasst, die den Lichtfluss des besagten zweiten Beleuchtungsmoduls (16) erhöhen können, wenn die Erfassungseinrichtungen (24) eine Zone geringer Beleuchtung erfassen.

2. Beleuchtungsvorrichtung nach Anspruch 1 **dadurch gekennzeichnet, dass** die Lichtquelle (12) die Überlappungszone (32) mit einer bestimmten Beleuchtungsstärke beleuchten kann und dass die Steuereinrichtungen (18, 22) den Lichtfluss des besagten zweiten Beleuchtungsmoduls (16) so erhöhen können, dass die Beleuchtung der Überlappungszone (32) gleich der bestimmten Beleuchtungsstärke ist.

3. Beleuchtungsvorrichtung nach Anspruch 1 oder 2 **dadurch gekennzeichnet, dass** die Erfassungseinrichtungen (24) eine Schattenzone (38) erfassen können, die durch ein Hindernis erzeugt wird, das sich im Überlappungsvolumen (42) des besagten ersten Beleuchtungsmoduls (16) befindet und wenigstens teilweise im Inneren der Überlappungszone (32) liegt, die Steuereinrichtungen (18, 22) den Lichtfluss des besagten zweiten Beleuchtungsmoduls (16) in einem bestimmten Maß erhöhen können und das bestimmte Maß gleich dem Verhältnis der Fläche der Schattenzone (38) zur Fläche der Überlappungszone (32) ist.

4. Beleuchtungsvorrichtung nach einem der Ansprüche 1 bis 3 **dadurch gekennzeichnet, dass** die Steuereinrichtungen (22) Einrichtungen zum Verkleinern des Lichtflusses umfassen, die den Lichtfluss des besagten ersten Beleuchtungsmoduls (16) herabsetzen können, wenn eine Zone geringer Beleuchtung durch die Erfassungseinrichtungen (24) festgestellt wird.

5. Beleuchtungsvorrichtung nach einem der vorhergehenden Ansprüche **dadurch gekennzeichnet, dass** der erste und der zweite Beleuchtungsmodul (16) jeweils Licht mit gleicher spektraler Zusammensetzung aussenden können.

6. Beleuchtungsvorrichtung nach einem der Ansprüche 1 bis 5 **dadurch gekennzeichnet, dass** wenigstens einer der Beleuchtungsmodule (16) eine Leuchtdiode (28) umfasst.

7. Beleuchtungsvorrichtung nach Anspruch 6 **dadurch gekennzeichnet, dass** wenigstens einer der Beleuchtungsmodule (16) mehrere Leuchtdioden (28) umfasst, von denen wenigstens zwei Leuchtdioden (28) Licht in verschiedener Farbe aussenden können, und dass die Steuereinrichtungen eine Steuereinheit (18) umfassen, die jedem Beleuchtungsmodul (16) zugeordnet ist, welche Steuereinheit (18) den individuellen Lichtfluss des Lichtes, das von jeder der Leuchtdioden (28) des Beleuchtungsmoduls (16) ausgesandt wird, in Abhängigkeit von einem gegebenen Sollwert regulieren kann, der eine Farbe und eine Lichtstärke wiedergibt.

8. Beleuchtungsvorrichtung nach Anspruch 7 **dadurch gekennzeichnet, dass** die Lichtquelle (12) eine Vielzahl von Beleuchtungsmodulen (16) umfasst, von denen jeder mehrere Leuchtdioden (28) umfasst, dass die Steuereinrichtungen eine Zentraleinheit (22) umfassen, die einzelne Sollwerte erzeugen kann, die eine Farbe und eine Lichtstärke für jeden der Beleuchtungsmodule (16) wiedergeben, und dass die Steuereinheit (18) mit der Zentraleinheit (22) so verbunden ist, dass sie die Sollwerte der Farbe und der Lichtstärke empfangen kann.

9. Beleuchtungsvorrichtung nach Anspruch 8 **dadurch gekennzeichnet, dass** wenigstens ein Beleuchtungsmodul (16) Einrichtungen (70) zum Erfassen einer fehlerhaften Leuchtdiode (28) umfasst und dass weiterhin Kompensationseinrichtungen für den Ausfall einer fehlerhaften Leuchtdiode vorgesehen sind.

10. Beleuchtungsvorrichtung nach Anspruch 9 **dadurch gekennzeichnet, dass** die Kompensationseinrichtungen Einrichtungen umfassen, die den Lichtfluss der Leuchtdioden (28) herabsetzen können, die das Licht einer anderen Farbe als der der fehlerhaften Leuchtdiode (28) aussenden.

11. Beleuchtungsvorrichtung nach Anspruch 9 **dadurch gekennzeichnet, dass** der Beleuchtungsmodul wenigstens eine weitere Leuchtdiode (28) einer Farbe, die gleich der der fehlerhaften Leuchtdiode ist, umfasst und dass die Kompensationseinrichtungen den Lichtfluss der Leuchtdiode mit der gleichen Farbe wie der der fehlerhaften Leuchtdiode (28) erhöhen können.

12. Beleuchtungsvorrichtung nach einem der Ansprüche 1 bis 11 **dadurch gekennzeichnet, dass** der Farbwiedergabeindex (CRI) der Lichtquelle (12) wenigstens gleich 90 ist.

13. Verwendung einer Beleuchtungsvorrichtung nach einem der vorhergehenden Ansprüche zum Beleuchten eines Operationsfeldes eines Arztes.

## Claims

1. A lighting device for a field of illumination (4), in particular that for a workspace used by a doctor, of a type which includes a light source (12) made up of first and second lighting modules (16), with these lighting modules (16) respectively being capable of illuminating first and second illuminated areas (30) of the field of illumination (4). The aforementioned illuminated areas (30) overlap at least partially to form an overlap zone (32), and each lighting module (16) defines an overlap volume (42) which extends between the surface of the lighting module (16) emitting light towards the overlap zone (32), and this overlap zone (32), **characterised by** the fact that it involves:
- means of detection (24) which are capable of detecting any less well illuminated areas in the overlap zone (32); and
- means of control (18, 22) that are capable of increasing the luminous flux of the aforementioned second lighting module (16) when the means of detection (24) detect a less well illuminated area.

2. A lighting device as described in claim 1, **characterised by** the fact that the light source (12) is capable of illuminating the overlap zone (32) at a given level of illumination, and by the fact that the means of control (18, 22) are capable of increasing the luminous flux of the aforementioned second lighting module (16) with the result that the illumination of the overlap zone (32) is equal to the pre-determined level of illumination.

3. Lighting device as described in claims 1 or 2, **characterised by** the fact that the means of detection (24) are capable of detecting a shadow zone (38) which is created by an obstacle located in the overlap volume (42) of the aforementioned lighting module (16) and which is located at least partially inside the overlap zone (32), by the fact that the means of control (18, 22) are capable of increasing the luminous flux of the aforementioned lighting module (16) by a pre-determined rate, and by the fact that the predetermined rate is equal to the ratio of the surface area of the shadow zone (38) to the surface area of the overlap zone (32).

4. A lighting device as described in any of claims 1 to 3 whatsoever, **characterised by** the fact that the means of control (22) include a means of reducing the luminous flux that is capable of reducing the luminous flux of the aforementioned first lighting module (16) when a less well illuminated zone is detected by the means of detection (24).

5. Lighting device as described in any of the preceding claims whatsoever and **characterised by** the fact that the aforementioned first and second lighting modules (16) are both capable of emitting light with an identical spectral composition.

6. A lighting device as described in any of claims 1 to 5 whatsoever, **characterised by** the fact that at least one of the lighting modules (16) includes a light emitting diode (28).

7. A lighting device as described in claim 6, **characterised by** the fact that at least one of the lighting modules (16) involves several light emitting diodes (28) of which light emitting diodes (28) at least two are capable of emitting light of different colours, and by the fact that the means of control involve a control unit (18) associated with each lighting module (16), with this control unit (18) being capable of adjusting the individual luminous flux of the light emitted by each of the light emitting diodes (28) in the lighting module (16), in accordance with a given setting value which corresponds to a colour and an intensity.

8. A lighting device as described in claim 7, **characterised by** the fact that the light source (12) is made up of multiple lighting modules (16) each involving several light emitting diodes (28), by the fact that the means of control include a central unit (22) which is capable of generating individual setting values which represent a colour and intensity of light for each lighting module (16), and by the fact that each control unit (18) is connected to a central unit (22) in such a way that they are able to receive the colour and intensity settings.

9. Lighting device as described in claim 8, **characterised by** the fact that at least one lighting module (16) includes means (70) of detecting a defective light emitting diode (28) and furthermore by the fact that it includes means for compensating for a fault in a defective light emitting diode.

10. Lighting device as described in claim 9, **characterised by** the fact that the means of compensation involve a suitable means of reducing the luminous flux from light emitting diodes (28) which are emitting light of a colour other than that of the defective light emitting diode (28).

11. A lighting device as described in claim 9, **characterised by** the fact that the lighting module includes at least one other light emitting diode (28) of a colour that is identical to that of the defective light emitting diode, and by the fact that the means of compensation are capable of increasing the luminous flux of the light emitting diode which has the same colour as the defective light emitting diode (28).

12. Lighting device as described in any of claims 1 to 11 whatsoever, **characterised by** the fact that the colour rendering index (CRI) for the light source (12) is at least 90.

13. The use of a lighting device as described in any of the preceding claims whatsoever in order to illuminate a doctor's workspace.
